# EUROPEAN PATENT SPECIFICATION

(11) **EP 0 340 068 B1**
(45) Date of publication and mention of the grant of the patent: **16.03.1994**
(21) Application number: 89401028.9
(22) Date of filing: 14.04.1989
(51) Int. Cl.: C12N 9/08, C12N 9/96

(54) **Use of para-amino-salicylic acid for the stabilization of the enzymatic activity of peroxidase in solution, stabilizing agent and stabilized solution in application thereof**
Verwendung von Paraaminosalicylsäure zur Stabilisierung der enzymatischen Aktivität von Peroxydase in Lösung, Stabilisierungsmittel und Verwendung von stabiliserter Lösung
Utilisation de l'acide para-amino-salicilique pour la stabilisation de l'activité enzymatique du peroxydase en solution, agent stabilisant et application de la solution stabilisée

(30) Priority: 20.04.1988 FR 8805243
(43) Date of publication of application: 02.11.1989
(73) Proprietor: ANDA BIOLOGICALS, F-67000 Strasbourg (FR)
(72) Inventor: ANDA BIOLOGICALS, F-67000 Strasbourg (FR)
(74) Representative: Portal, Gérard

(56) References cited:
- EP-A- 0 196 518
- EP-A- 0 197 447

## Description

The present invention concerns the use of para-amino-salicylic acid for the stabilization of the enzymatic activity of peroxidase in solution, stabilizing agent and stabilized solution in application thereof.

Peroxidase is a frequently used enzyme in diagnostic tests based on enzyme-immunological reactions. Among these, one may mention the pregnancy tests and tests for the detection of antiviral antibodies, such as hepatitis, human deficiency virus and rubella.

Peroxidase, coupled or not to immunological reagents, is unstable in diluted form. This obliges the test manufacturer to furnish the enzyme in a concentrated form in a liquid medium containing stabilizing agents such as bovine albumin or serum, whose concentrations may be as high as 20 % of the total reagent volume. The user is therewith forced to proceed to a dilution of the reagent at the moment of use, which may bring about sources of error and additional costs.

It is therefore advantageous to find stabilizing substances that allow the presentation of peroxidase-coupled reagents under a diluted form, ready-to-use, bringing about the additional requirement to sometimes work at a pH near neutrality.

Various investigations have been made, resulting in patents :
- DE 35 09 238 from BOEHRINGER Mannheim
- DE 35 11 327 also from BOEHRINGER, advocating the incorporation of phenol or amino-pyrine at a concentration equal or superior to 0.005 %.
- CH 4944/84 from HOFFMANN-LA ROCHE, claiming 4-amino-antipyrine
- EP-A-0,110,429 from ABBOTT, advocating the use of anilino-8-naphtalene-sulfonic acid.
None of these substances is satisfactory.

The aim of this invention is to solve the new technical problem consisting in the production of a new stabilizing agent of the enzymatic activity of peroxidase solutions, either at 4°C or at room temperature, for at least 10 days.

This new technical problem is now solved for the first time by the present invention. It has indeed been found that para-amino-salicylic acid (PASA) is endowed with remarkable stabilizing properties of the enzymatic activity of peroxidase in solution, either at low temperatures or at 37°C during at least 10 days.

This is quite surprising inasmuch that a derivative of salicylic acid, 5-amino-salicylic acid, is a substrate of enzymatic reaction for peroxidase, making it unexpected to find among the derivatives of salicylic acid a compound able to stabilize the enzymatic activity of peroxidase.

In addition, as it will be demonstrated from examples, other derivatives of salicylic acid have no stabilizing effect on the enzymatic activity of peroxidase.

The invention thus concerns the use of para-amino-salicylic acid as a stabilizing agent of the enzymatic activity of peroxidase in solution, free or coupled to an immunological reagent.

A second aspect of the invention concerns the stabilizing agent itself, containing the PASA. An advantageous characteristic is to set the concentration of PASA between about 8 µg/ml and about 2,000 µg/ml (microgramme/millilitre) of the solution of stabilizing agent or of the solution to stabilize.

This stabilizing agent contains advantageously para-amino-salicylic acid solubilized in a buffer constituted of Tris brought to a pH comprised between about 7 and 8 with an acid chosen preferentially among acetic acid, succinic acid, boric acid and diethylbarbituric acid.

This stabilizing agent may in addition contain at least 2 % serum.

The present invention concerns finally a solution of stabilized peroxidase, characterized in that it contains an effective amount of para-amino-salicylic acid for the stabilization. Preferentially, this effective amount ranges between about 8 µg/ml and about 2,000 µg/ml of the stabilized solution.

This solution of stabilized peroxidase is preferentially in a Tris buffer. Preferentially, this solution contains also at least 2 % serum. According to another embodiment, this solution of stabilized peroxidase contains at least 0.01 % in weight of CaCl₂ and at least 0.01 % in weight of MgCl₂ , 6H₂O.

According to a further embodiment, this stabilized solution is under a ready for use form with a concentration in peroxidase equal or inferior to 50 µg/ml of the total solution.

Finally, the invention concerns also the process of preparation of the stabilizing agent and of the solution of stabilized peroxidase that results directly for the man skilled in the art from what has been said as well as from the descriptions that follow by introducting or incorporating para-amino-salicylic acid into said stabilizing agent or said peroxidase solution.

Other purposes, characteristics and advantages will appear at the light of the description that follows, made from the following examples that may not be in any way construed to limit the scope of this invention.

### EXAMPLE 1

4-amino-salicylic acid, also called para-amino-salicylic acid (PASA), sulfo-salicylic acid and 5-nitro-salicylic acid were solubilized at a concentration of 2 mg/ml in a solution 0.02 M. Tris. The pH was adjusted to 8 with 0.025 M. acetic acid and to 7 with 0.025 M. succinic acid, except for sulfo-salicylic acid, which is acid.

Peroxidase was added to these media and the solutions were kept at 4°C and at 37°C during 11 days. The residual enzymatic activity was measured by the introduction of 5 µl of each solution into 100 µl of a substrate of enzymatic reaction consisting in 0.4 ml ortho-phenylene diamine (OPD) solubilized in 1 ml of a citric acid-phosphate buffer at pH 5.3 containing 0.02 % H₂O₂. After 10 minutes of incubation, the yellow colored reaction was stopped by the addition of 100 µl of 4 N H₂SO₄ and the intensity of the coloration was measured at 492 nm. The results of this experiment are assembled in table 1.

**Table 1**

| | control | | acetic acid | | succinic acid | |
|---|---|---|---|---|---|---|
| | 37° | 4° | 37° | 4° | 37° | 4° |
| Peroxidase control. | 1.29 | 2.17 | 0.54 | 2.16 | 0.10 | 0.77 |
| PASA (invention) | 1.66 | 2.42 | 1.75 | 2.05 | 1.19 | 1.88 |
| Sulfo-salicyl. | 0.13 | 2.20 | 0.83 | 2.22 | 0.15 | 1.50 |
| Nitro-salicyl. | 0.24 | 1.88 | 0.73 | 2.02 | 0.15 | 1.20 |

The superiority of PASA is evident at 4°C and 37°C and also when the pH of the solution is brought down to 8 or 7.

On the contrary, sulfo-salicylic and nitro-salicylic acid show no significant stabilizing activity versus the control.

### EXAMPLE 2

The optimal concentration of PASA was thereafter determined. Peroxidase was dissolved in a buffer Tris-barbituric acid in the presence of various concentrations of PASA during 9 days at 4°C and 37°C. The residual enzymatic activity was analyzed as per example 1 and the results reported in table 2.

**Table 2**

| PASA concentration | 37°C | 4°C |
|---|---|---|
| 0 µg/ml | 0.40 | 2.0 |
| 8 | 0.79 | 1.97 |
| 40 | 1.11 | 2.0 |
| 200 | 1.52 | 2.0 |
| 1,000 | 1.65 | 2.0 |

The PASA concentration is in µg/ml and the optical density were read at 292 nm. It is clear from the results here shown that the stabilizing effect of PASA takes place at concentrations as low as 8 µg/ml but is optimal from 200 µg/ml on.

### EXAMPLE 3

A solution of peroxidase that was 0.6 % in Tris, was made 0.4 % in boric acid and 0.05 % in PASA. This solution was supplemented with 2 % turkey serum and the two solutions so obtained were incubated at 4°C and 37°C during 7 days, after which an analysis was made of the residual enzymatic activity. Results are given in table 3.

**Table 3**

| Control | | Serum | |
|---|---|---|---|
| 37°C | 4°C | 37°C | 4°C |
| 0.4 | 1.38 | 1.40 | 1.59 |

One sees that the enzymatic activity of the peroxidase solution is maintained at 37°C in the presence of serum.

### EXAMPLE 4

Anilino-8-naphtalene-1-sulfonic acid (ANSA), phenol, aminopyrine and 4-amino-antipyrine were applied according to the recommendations given in the patents and compared to the enzymatic acitivity of peroxidase solubilized in a medium that was 0.9 % in NaCl, buffered at pH 8.0 with Tris -acetic acid 0.05 M., containing 5 % serum, 0.25 % PASA and 0.1 % CaCl₂-MgCl₂ , 6H₂O.

The solution formed a precipitate that was discarded through filtration, before the addition of the enzyme . The solutions were placed at 37°C and 4°C during 7 days and analyzed according to example 1. Results are given in table 4.

**Table 4**

| | Invention | Previous art | | | |
|---|---|---|---|---|---|
| | PASA | Aminopyrine | Phenol | Antipyrine | ANSA. |
| 37°C | 1.78 | 0.4 | 0.0 | 0.55 | 1.1 |
| 4°C | 1.73 | 1.5 | 1.2 | 1.40 | 1.5 |

PASA is superior to the other substances tested. This stability of the enzyme is also observed at 4°C and remained fully present at 37°C, on the contrary to what is observed with the substances advocated in the previous art.

## Claims

1. Use of para-amino-salicylic acid for the stabilization of the enzymatic activity of peroxidase in solution, free or coupled to an immunologivcal reagent.

2. Stabilizing agent of the enzymatic activity of peroxidase in solution, characterized in that it contains para-amino-salicylic acid.

3. Stabilizing agent according to claim 2, characterized in that it contains para-amino-salicylic acid in a quantity adequate for the stabilization, preferentially comprised, between about 8 µg/ml and about 2,000 µg/ml of the solution of stabilizing agent or of the solution to stabilize.

4. Stabilizing agent according to claim 2 or 3, characterized in that para-amino-salicyclic acid is solubilized in a Tris buffer brought to a pH comprised between about 7 and 8 with an acid preferentially chosen between acetic acid, succinic acid, boric acid and diethylbarbituric acid.

5. Stabilizing agent according to claims 2 to 4, characterized in that it contains at least 2 % serum.

6. Solution of stabilized peroxidase, characterized in that it contains an effective amount of para-amino-salicylic acid for stabilization.

7. Solution of stabilized peroxidase according to claim 6, characterized in that the effective amount for stabilization ranges between about 8 µg/ml and 2,000 µg/ml of stabilized solution.

8. Solution of stabilized peroxidase according to claims 6 and 7, characterized in that the solution is in Tris buffer.

9. Solution of stabilized peroxidase according to anyone of claims 6 to 8, characterized in that it contains also 2% serum.

10. Solution of peroxidase according to anyone of claims 6 to 9, characterized in that it contains at least 0.01 % in weight of CaCl₂ and at least 0.01 % in weight of MgCl₂, 6H₂O.

11. Solution of peroxidase according to anyone of claims 6 to 10, characterized in that it is under a ready-to-use form with a concentration in peroxidase equal or inferior to 50 µg/ml of the total solution.

## Patentansprüche

1. Verwendung von p-Aminosalicylsäure zur Stabilisierung der enzymatischen Aktivität von Peroxidasen in Lösung, die frei vorliegen oder an ein immunologisches Reagens gekoppelt sind.

2. Stabilisierungsmittel zur Stabilisierung der enzymatischen Aktivität von Peroxidasen in Lösung, dadurch gekennzeichnet, daß es p-Aminosalicylsäure enthält.

3. Stabilisierungsmittel nach Anspruch 2, dadurch gekennzeichnet, daß es p-Aminosalicylsäure in einer für die Stabilisierung ausreichenden Menge enthält, die bevorzugt zwischen etwa 8 und etwa 2000 µg pro Milliliter der Lösung des Stabilisierungsmittels oder der zu stabilisierenden Lösung liegt.

4. Stabilisierungsmittel nach Anspruch 2 oder 3, dadurch gekennzeichnet, daß die p-Aminosalicylsäure in einem Tris-Puffer gelöst ist, der mit einer Säure auf einen pH-Wert zwischen 7 und 8 eingestellt ist, die bevorzugt unter Essigsäure, Bernsteinsäure, Borsäure und Diethylbarbitursäure ausgewählt ist.

5. Stabilisierungsmittel nach den Ansprüchen 2 bis 4, dadurch gekennzeichnet, daß es mindestens 2 % Serum enthält.

6. Lösungen stabilisierter Peroxidasen, dadurch gekennzeichnet, daß sie eine wirksame Menge p-Aminosalicylsäure zur Stabilisierung enthalten.

7. Lösungen stabilisierter Peroxidasen nach Anspruch 6, dadurch gekennzeichnet, daß die wirksame Menge zur Stabilisierung im Bereich zwischen etwa 8 und 2000 µg pro Milliliter der stabilisierten Lösung liegt.

8. Lösungen stabilisierter Peroxidasen nach Anspruch 6 und 7, dadurch gekennzeichnet, daß die Lösung in einem Tris-Puffer vorliegt.

9. Lösungen stabilisierter Peroxidasen nach einem der Ansprüche 6 bis 8, dadurch gekennzeichnet, daß sie ferner 2 % Serum enthalten.

10. Lösungen von Peroxidasen nach einem der Ansprüche 6 bis 9, dadurch gekennzeichnet, daß sie mindestens 0,01 Gew.-% CaCl₂ und mindestens 0,01 Gew.-% MgCl₂, 6H₂O enthalten.

11. Lösungen von Peroxidasen nach einem der Ansprüche 6 bis 10, dadurch gekennzeichnet, daß sie in gebrauchsfertiger Form mit einer Peroxidasekonzentration ≦ 50 µm pro Milliliter der Gesamtlösung vorliegen.

## Revendications

1. Utilisation de l'acide para-aminosalicylique pour la stabilisation de l'activité enzymatique de la peroxydase en solution, libre ou couplée avec un réactif immunologique.

2. Agent de stabilisation de l'activité enzymatique de la peroxydase en solution, caractérisé en ce qu'il contient l'acide para-aminosalicylique.

3. Agent de stabilisation selon la revendication 2, caractérisé en ce qu'il contient l'acide para-aminosalicylique en une quantité appropriée dans un but de stabilisation, de préférence comprise entre environ 8 µg/ml et environ 2000 µg/ml de la solution d'agent stabilisant ou de la solution à stabiliser.

4. Agent de stabilisation selon la revendication 2 ou 3, caractérisé en ce que l'acide para-aminosalicylique est dissous dans un tampon Tris ramené à un pH compris entre environ 7 et 8 par un acide de préférence choisi parmi l'acide acétique, l'acide succinique, l'acide borique et l'acide diéthylbarbiturique.

5. Agent de stabilisation selon les revendications 2 à 4, caractérisé en ce qu'il contient au moins 2 % de sérum.

6. Solution de peroxydase stabilisée, caractérisée en ce qu'elle contient une quantité efficace d'acide para-aminosalicylique dans un but de stabilisation.

7. Solution de peroxydase stabilisée selon la revendication 6, caractérisée en ce que la quantité efficace dans un but de stabilisation est comprise entre environ 8 µg/ml et environ 2 000 µg/ml de solution stabilisée.

8. Solution de peroxydase stabilisée selon les revendications 6 et 7, caractérisée en ce que la solution est dans un tampon Tris.

9. Solution de peroxydase stabilisée selon l'une quelconque des revendications 6 à 8, caractérisée en ce qu'elle contient en outre 2 % de sérum.

10. Solution de peroxydase selon l'une quelconque des revendications 6 à 9, caractérisée en ce qu'elle contient au moins 0,01 % en poids de CaCl₂ et au moins 0,01 % en poids de MgCl₂,6H₂O.

11. Solution de peroxydase selon l'une quelconque des revendications 6 à 10, caractérisée en ce qu'elle est sous une forme prête à l'emploi avec une concentration en peroxydase inférieure ou égale à 50 µg/ml de la solution totale.
